# EUROPÄISCHE PATENTSCHRIFT

(11) **EP 2 257 271 B1**
(45) Veröffentlichungstag und Bekanntmachung des Hinweises auf die Patenterteilung: **29.03.2017**
(21) Anmeldenummer: 09728521.7
(22) Anmeldetag: 03.02.2009
(51) Int. Cl.: A61K 8/73, A61K 8/11, A61K 8/19, A61K 8/22, A61K 8/23, A61K 8/24, A61K 8/25, A61Q 5/08

(54) **MITTEL ZUM AUFHELLEN VON KERATINHALTIGEN FASERN MIT BESCHICHTETEN PARTIKELN**
AGENTS FOR LIGHTENING FIBERS CONTAINING KERATIN HAVING COATED PARTICLES
AGENT DE DÉCOLORATION DE FIBRES KÉRATINIQUES CONTENANT DES PARTICULES REVÊTUES

(30) Priorität: 03.04.2008 DE 102008017439
(43) Veröffentlichungstag der Anmeldung: 08.12.2010
(73) Patentinhaber: Henkel AG & Co. KGaA, 40589 Düsseldorf (DE)
(72) Erfinder: MANNECK, Hartmut, 23860 Klein Wesenberg (DE); KLEEN, Astrid, 20457 Hamburg (DE); AKRAM, Mustafa, 22455 Hamburg (DE); WELZ, Carolin, 22459 Hamburg (DE)
(86) Internationale Anmeldenummer: PCT/EP2009/051176
(87) Internationale Veröffentlichungsnummer: WO 2009/121643

(56) Entgegenhaltungen:
- EP-A1- 1 228 763
- EP-A1- 1 752 191
- DE-A1- 1 617 826
- DE-A1- 3 801 606
- GB-A- 2 130 087
- LU-A1- 57 898
- US-A1- 2008 031 940
- K.H. Bauer, K.-H. Frömming, C. Führer: "Lehrbuch der Pharmazeutischen Technologie" 1999, Wissenschaftliche Verlagsgesellschaft mbH , Stuttgart , XP002592689 ISBN: 3-8047-1700-4 , Seiten 329-337 Tabelle 14.4 Seite 330; Abbildung 14.31

## Beschreibung

Die Erfindung betrifft Mittel zum Aufhellen von keratinhaltigen Fasern, insbesondere Blondiermittel für menschliche Haare.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die so genannten Blondierverfahren, im Wesentlichen nur dunkelblonde oder hellere Haare. Die Grundlagen der Blondierverfahren sind dem Fachmann bekannt und können in einschlägigen Monographien, z.B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

So werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung mit einer Wasserstoffperoxidlösung vermischt werden, werden im Weiteren als "Blondiermittel" bzw. "Blondierpulver" bezeichnet. Alle aufgeführten Mengenangaben beziehen sich, soweit nicht anders ausgeführt, ausschließlich auf diese Zubereitungen.

Die vorliegende Erfindung bezieht sich auch auf Mittel zum Aufhellen von keratinhaltigen Fasern, insbesondere menschlichen Haaren.

In Blondiermitteln besteht das Problem, dass ein Oxidationsmittel, das dem Mittel zugesetzt wird, um ein Anwendungsprodukt zu erhalten, schnell zersetzt wird. Diese Zersetzungsreaktion wird insbesondere durch Alkalisierungsmittel beschleunigt. Bei Zersetzung von Wasserstoffperoxid tritt eine Gasentwicklung ein. Diese kann während der Handhabung der Blondiermittel je nach Intensität ein Gefahrenpotenzial für den Anwender in sich bergen.

Zudem werden Blondiermittel oft dazu benutzt, nur einzelne Haarsträhnen aufzuhellen, um eine optisch interessante Haarfärbung zu erzeugen. Beim herkömmlichen "Strähnchenprozess" werden einzelnen Haarsträhnen in Folien eingelegt, mit der Blondiermittelzubereitung beaufschlagt und in die Folie eingeschlagen. Da der Applikationsprozess langwierig ist, sind die Einwirkzeiten auf die nacheinander beaufschlagten Strähnen unterschiedlich, was zu unerwünschten weil unterschiedlichen Färbeergebnissen mit verschieden hellen Strähnen führt.
Daher ist eine größere zeitliche Unabhängigkeit für den Anwender, insbesondere für den professionellen Anwender, beim Auftragen und Ausspülen der Blondiermittel wünschenswert.

Der vorliegenden Erfindung lag die Aufgabe zugrunde, Mittel zum Aufhellen von Keratinfasern bereitzustellen, die es ermöglichen, auch bei der "Strähnchentechnik" ein einheitliches Färbeergebnis zu erzielen. Das Überbleichen einzelner Strähnen, die im Prozess vor anderen Strähnen mit der Applikationsmischung beaufschlagt werden, sollte vermieden werden.

GB 2130087 A offenbart pharmazeutische, oral verabreichbare Wirkstoffe, die geeignet sind Hydrogencarbonat in einem Säugetierkörper frei zu setzen. Die Beispiele der D1 offenbaren Tabletten, bei denen Natriumhydrogencarbonat-Zubereitungen von bestimmten Cellulose-Hüllen umgeben sind.

EP 1228763 A1 offenbart Partikel, die von bestimmten Cellulosen umhülltes Lithiumcarbonat umfassen.

US 2008/0831940 beschreibt Tabletten, bei denen u.a. Calciumphosphat von Ethylcellulose umhüllt ist.

EP 1752191 A1 offenbart Mittel zum Aufhellen und/oder Färben von Keratinfasern, welche ein umhülltes Alkalisierungsmittel enthalten, wobei als mögliches Hüllmaterial Fettsäuren, Lactone, Säureanhydride und wasserlösliche Polymere genannt werden In dem offenbarten Beispiel der Schrift wird mit Stearinsäure- bzw. Isostearinsäure-Gemisch überzogenes Natriumsilikat mit einer Wasserstoffperoxid-Lösung versetzt.

DE 1617826 offenbart Mittel zum Färben und Blondieren von Haaren, welches mikroverkapselte Oxidationsfarbstoffvorprodukte und/oder mikroverkapselte Wasserstoffperoxid-Addukte sowie gegebenenfalls mikroverkapselte Alkalisierungsmittel enthält. Als Verkapselungsmaterial wird u.a. Celluloseacetatphthalat genannt.

LU 57898 A1 offenbart Aufhellmittel für Haare, enthaltend mikroverkapselte Inhaltsstoffe, unter anderem verkapseltes Natriummetasilikat und mikroverpaselte Persulfatsalze. Als Verkapselungsmaterial ist Cellulosephthalatacetat genannt.

DE 3801606 A1 adressiert oxidative Färbemittel, welche mikroverkapselte Acidificierungsmittel enthalten. Beispielhaft offenbart wird eine Wasserstoffperoxidzubereitung, welche in Cellulosephthalatacetat mikroverkapselte Polyacrylsäure enthält.

Es wurde nun gefunden, dass sich die vorstehenden Aufgaben lösen lassen, indem Blondiermittel bereitgestellt werden, in denen mindestens ein partikelförmiger Bestandteil enthalten ist, welcher eine Beschichtung aus bestimmten Stoffen aufweist. Besonders bevorzugte Ausführungsformen der vorliegenden Erfindung sehen vor, dass das Blondiermittel vollständig aus partikelförmigen Bestandteilen besteht, welche alle beschichtet sind.

Durch die Beschichtung des Blondiermittels wird der Start des eigentlichen Blondiervorgangs verzögert, und somit eine größere zeitliche Unabhängigkeit beim Auftragen der Blondierung für den Anwender erreicht. Auch im Falle einer schlechten Zeiteinhaltung seitens des Anwenders beim Ausspülen der Strähnen wird daher eine gleichmäßige und damit schonende Blondierung erhalten. Nachfolgend werden die erfindungsgemäßen Mittel zum Aufhellen von Keratinfasern auch als "Blondiermittel", als "Aufhellmittel" bezeichnet. Diese Begriffe sind dabei nicht als beschränkend zu verstehen.
In einer ersten Ausführungsform betrifft die vorliegende Erfindung Mittel zum Aufhellen von keratinischen Fasern, insbesondere menschlicher Haare, enthaltend mindestens einen umhüllten Partikel und eine Oxidationsmittellösung, vorzugsweise eine Wasserstoffperoxidlösung, wobei die Partikel einen festen Partikelkern, ausgewählt aus Alkalimetall-silikaten, umfassen und eine diesen Kern umgebende Hülle beinhalten, dadurch gekennzeichnet, dass die Hülle zu mindestens 50 Gew.-% ihres Gewichts aus einer modifizierten Cellulose mit Monomereinheiten entsprechend Formel (I) 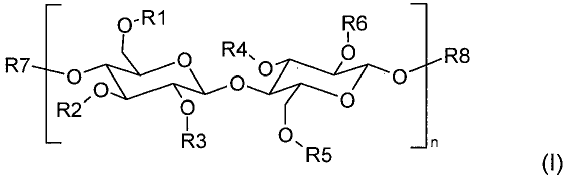 besteht,
worin R1 bis R8 unabhängig voneinander für Wasserstoff, einen linearen oder verzweigten C1 bis C6-Alkylrest, einen linearen oder verzweigten C1 bis C6-Hydroxyalkylrest, enthaltend eine oder mehrere Hydroxylgruppen, einen linearen oder verzweigten C1 bis C6-Alkoxyalkylrest, enthaltend eine oder mehrere C1 bis C4-Alkoxygruppen, einen linearen oder verzweigten C1 bis C6-Acylrest oder einen gegebenenfalls substituierten Benzoylrest steht, mit der Maßgabe, dass
- mindestens einer der Reste R1 bis R6 ungleich Wasserstoff ist,
- R1 bis R6 unabhängig voneinander für Wasserstoff, einen Acetylrest oder einen Phthalatrest steht,
- mindestens einer der Reste R1 bis R6 für einen Acetylrest und mindestens einer der Reste R1 bis R6 für einen Phthalatrest steht.
- und n für eine ganze Zahl zwischen 10 und 1.000.000 steht.

In einer bevorzugten Ausführungsform der vorliegenden Erfindung besteht die den Partikelkern umgebende Hülle zu mindestens 70 Gew.-%, weiter bevorzugt zu mindestens 90 Gew.-% und insbesondere zu 100 Gew.-% ihres Gewichts aus modifizierter Cellulose mit Monomereinheiten entsprechend Formel (I).

Es ist erfindungsgemäß, Vertreter aus bestimmten Stoffgruppen als Partikelkern zu beschichten. Alkalisierungsmittel werden erfindungsgemäß in Form beschichteter Partikel eingesetzt.

Das Alkalisierungsmittel dient zur Einstellung eines alkalischen pH-Wertes der Anwendungsmischung. Erfindungsgemäß können Alkalimetall-silikate verwendet werden.

Bevorzugt wird das Beschichtungsmaterial in einer bestimmten Menge auf die zu beschichtenden Partikelkerne aufgebracht. Hier ist es erfindungsgemäß bevorzugt, dass die Hülle 10 bis 70 Gew.-% des Gewichts der beschichteten Partikel ausmacht. Die Beschichtungsmaterialien werden nachstehend beschrieben.

Erfindungsgemäße Beschichtungsmaterialien für die zu beschichteten Partikelkerne sind modifizierte Cellulosen mit Monomereinheiten entsprechend Formel (I) 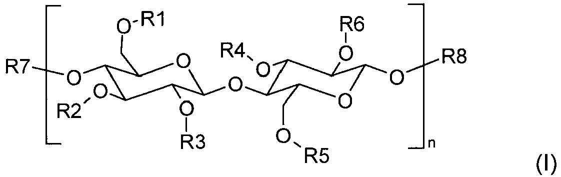 besteht,
worin R1 bis R8 unabhängig voneinander für Wasserstoff, einen linearen oder verzweigten C1 bis C6-Alkylrest, einen linearen oder verzweigten C1 bis C6 Hydroxyalkylrest, enthaltend eine oder mehrere Hydroxylgruppen, einen linearen oder verzweigten C1 bis C6 Alkoxyalkylrest, enthaltend eine oder mehrere C1 bis C4 Alkoxygruppen, einen linearen oder verzweigten C1 bis C6 Acylrest oder einen gegebenenfalls substituierten Benzoylrest steht, mit der Maßgabe, dass mindestens einer der Reste R1 bis R6 ungleich Wasserstoff ist,
- R1 bis R6 unabhängig voneinander für Wasserstoff, einen Acetylrest oder einen Phthalatrest steht,
- mindestens einer der Reste R1 bis R6 für einen Acetylrest und mindestens einer der Reste R1 bis R6 für einen Phthalatrest steht.
und n für eine ganze Zahl zwischen 10 und 1.000.000 steht.

Erfindungsgemäß sind solche Partikel, deren Hülle mindestens eine modifizierte Cellulose gemäß Formel (I) enthält, worin R1 bis R6 unabhängig voneinander für Wasserstoff, einen Acetylrest oder einen Phthalatrest (2-Hydroxycarbonyl-benzoylrest) steht, mit der Maßgabe, dass mindestens einer der Reste R1 bis R6 für einen Acetylrest und mindestens einer der Reste R1 bis R6 für einen Phthalatrest (2-Hydroxycarbonyl-benzoylrest) steht. Eine solchermaßen modifizierte Cellulose wird als 30 Gew.-% wässrige Dispersion unter dem Handelsnamen Aquacoat^{®} CPD von der Firma FMC BioPolymer vertrieben.

Die erfindungsgemäßen beschichteten Partikel besitzen einen mittleren Teilchendurchmesser von 50 bis 500 µm, bevorzugt von 100 µm bis 250 µm.

Sofern die Hülle nicht zu 100 % aus den genannten Substanzen besteht, kann sie weitere Inhaltsstoffe wie Farb- und Duftstoffe oder Hilfsstoffe enthalten.

Besonders bevorzugte erfindungsgemäße Partikel enthalten in der Hülle Weichmacher für eine bessere Elastizität der Hülle. Weichmacher stammen bevorzugt aus der Gruppe Dialkylphthalat, insbesondere Diethylphthalat, Triethylcitrat, Glycerintriacetat und/oder der Polyethylenglycole.

In einer weiteren Ausführungsform enthalten die erfindungsgemäßen beschichteten Partikel in der den Partikelkern umgebenden Hülle zusätzlich mindestens ein Desintegrationsmittel.

Derartige Desintegrationsmittel werden in der Literatur häufig auch als Zerfallsmittel oder Sprengmittel beschrieben. Derartige Substanzen werden in die Polymerumhüllung eingearbeitet, um deren Zerfallszeiten zu verkürzen. Dieser Zerfall oder diese Sprengung geschieht insbesondere durch eine Volumenvergrößerung infolge von Wasserzutritt (Quellung).

Gemäß Römpp (9. Auflage, Bd. 6, S. 4440) und Voigt "Lehrbuch der pharmazeutischen Technologie" (6. Auflage, 1987, S. 182-184) werden unter solchen Desintegrationsmitteln bzw. Zerfallsbeschleunigern Hilfsstoffe verstanden, die für den raschen Zerfall von Formkörpern in Wasser oder Magensaft und für die Freisetzung der eingeschlossenen Pharmaka in resorbierbarer Form sorgen.

Als bevorzugte Desintegrationsmittel werden im Rahmen der vorliegenden Erfindung Desintegrationsmittel auf Cellulosebasis eingesetzt. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxy-Gruppengegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen. Die genannten Cellulosederivate werden vorzugsweise nicht als einzige Desintegrationsmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist. Geeignete Carboxymethylcellulose-derivate werden beispielsweise unter dem Handelsnamen Tylopur^{®} von der Firma Clariant oder Ac-Di-Sol^{®} der Firma FMC vertrieben.

Als weiteres Desintegrationsmittel auf Cellulosebasis oder als Bestandteil dieser Komponente kann mikrokristalline Cellulose verwendet werden. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 µm aufweisen und beispielsweise zu Granulaten mit einer mittleren Teilchengröße von 200 µm kompaktierbar sind. Geeignete mikrokristalline Cellulose ist beispielsweise unter den Handelsnamen Emcocel^{®} von der Firma JRS Pharma oder Avicel^{®} von der Firma FMC kommerziell erhältlich.

Weiterhin kann bevorzugt auch Stärke als Desintegrationsmittel im Rahmen der vorliegenden Erfindung eingesetzt werden. Die erfindungsgemäß einsetzbare Stärke wird üblicherweise aus pflanzlichen Rohstoffen, wie Reis, Soja, Kartoffeln oder Mais gewonnen. Stärke kann unmodifiziert oder analog zur Cellulose als modifizierte Stärke eingesetzt werden. Besonders bevorzugte Stärke-Modifikationen liefern dabei Veresterungs- und Veretherungsreaktionen, insbesondere die erhaltenen Ether aus Reaktionen mit Hydroxycarbonsäuren. Eine erfindungsgemäß besonders geeignete Stärkemodifikation ist die Mischung aus Natriumcarboxymethylstärke und Natriumglykolstärke, die unter dem Handelsnamen Explotab^{®} durch die Firma JRS Pharma vertrieben wird.

Erfindungsgemäß besonders bevorzugt sind Desintegrationsmittel auf Maisstärke-Basis. Geeignete, modifizierte Maisstärken sind beispielsweise unter den Handelsnamen Glycolys^{®} von der Firma Roquette oder Starch 1500^{®} von der Firma Colorcon erhältlich.

Schließlich stellen Desintegrationsmittel aus vernetztem, wasserunlöslichen Polyvinylpyrrolidinon (PVP) eine weitere Klasse erfindungsgemäß besonders geeigneter Desintegrationsmittel dar. Die vorteilhafte Vernetzung dieser PVP-Modifikation beruht dabei vornehmlich auf Verwicklungen und Verschlingungen der einzelnen Polymerstränge ineinander. Ein erfindungsgemäß besonders bevorzugtes Desintegrationsmittel auf PVP-Basis wird unter dem Handelsnamen Kollidon^{®} CL durch die Firma BASF vertrieben.

Die erfindungsgemäßen, den Partikelkern umgebenden Beschichtungen enthalten die Zerfallshilfsmittel insbesondere in Mengen von 0,5 bis 20 Gew.-%, bevorzugt von 1 bis 10 Gew.-%, jeweils bezogen auch das Gesamtgewicht der getrockneten Hülle.

Weiterhin ist es erfindungsgemäß bevorzugt, wenn die den Partikelkern umgebende Hülle zusätzlich mindestens ein Trennmittel und/oder mindestens einen Porenbildner enthält

In einer bevorzugten Ausführungsform enthalten die erfindungsgemäßen beschichteten Partikel, in der den Partikelkern umgebenden Hülle zusätzlich mindestens ein Trennmittel.

Als Trennmittel haben sich insbesondere Talkum, amorphes Siliciumdioxid, welches beispielsweise unter dem Handelsnmaen Syloid^{®} 244FP von der Firma Grace GmbH vertrieben wird, Glycerinmonostearat, Magnesiumstearat und Silikate wie Aerosil bewährt. Bevorzugt enthält die Hülle als Trennmittel Talkum.

In einer weiteren Ausführungsform enthalten die erfindungsmäßen beschichteten Partikel in der den Partikelkern umgebenden Hülle zusätzlich mindestens einen Porenbildner.

Porenbildner werden in die Beschichtung mit eingearbeitet und bewirken, dass sich in der Oberfläche der Beschichtung Poren bilden: Dadurch kommt es zu einer Steigerung der Diffusionsrate in die Polymerhülle für hydrophile Substanzen, insbesondere Wasser.

Erfindungsgemäß eignen sich als Porenbildner besonders Polyvinylpyrrolidinon, Zucker und Zuckeralkohole, wie Lactose, Saccharose, Sorbit und Mannit, Polyethylenglykole mit weniger als 600 Ethylenoxideinheiten, sowie Cellulosederivate, wie Hydroxypropylcellulose, Hydroxypropylmethylcellulose, Methylcellulose und deren Mischungen.

Erfindungsgemäß besonders bevorzugte Porenbildner sind Polyvinylpyrrolidinone (PVP), die beispielsweise unter dem Handelsnamen Kollidon® von der Firma BASF vertrieben werden.

Erfindungsgemäß ist der Gewichtsanteil der Porenbildner in getrockneten Hülle zwischen 0,5 und 50 Gew.-%, insbesondere zwischen 1 und 30 Gew.-%, jeweils bezogen auf das Gesamtgewicht der Hülle.

Der Gegenstand der vorliegenden Erfindung betrifft ein Mittel zu Aufhellen von keratinischen Fasern, insbesondere menschlicher Haare, welches mindestens beschichtete Partikel gemäß der vorangegangenen Beschreibung enthält.

Die erfindungsgemäßen Mittel enthalten Alkalisierungsmittel (berechnet als unbeschichtetes Alkalisierungsmittel) bevorzugt in Mengen von 1 bis 50 Gew.-%, insbesondere 15 bis 40 Gew.-%, jeweils bezogen auf das gesamte Mittel.

Weiterhin hat es sich als vorteilhaft erwiesen, wenn die erfindungsgemäßen Blondiermittel nichtionogene grenzflächenaktive Stoffe enthalten. Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, dass die gewählten Substanzen bevorzugt frei von Lösungsmitteln, Stellmitteln und anderen Begleitstoffen sein sollen.

Bevorzugte nichtionogene grenzflächenaktive Stoffe sind
- alkoxylierte Fettalkohole mit 8 bis 22, insbesondere 10 bis 16, Kohlenstoffatomen in der Fettalkylgruppe und 1 bis 30, insbesondere 1 bis 15, Ethylenoxid- und/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal^{®}B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C1-C4-Alkyl-Ethers, "verschlossen". Ein Beispiel für eine solche Verbindung ist das Handelsprodukt Dehypon^{®}LT 054, ein C₁₂₋₁₈-Fettalkoholol + 4,5 Ethylenoxid-butylether.
- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure.
- alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.
- Polyglycerinester und alkoxylierte Polyglycerinester. Bevorzugte Verbindungen dieser Klasse sind beispielsweise Poly(3)glycerindiisostearat (Handelsprodukt: Lameform^{®}TGI (Henkel)) und Poly(2)glycerinpolyhydroxystearat (Handelsprodukt: Dehymuls^{®}PGPH (Henkel)).
- Sorbitan-Fettsäureester und alkoxylierte Sorbitan-Fettsäureester wie beispielsweise Sorbitanmonolaurat und Sorbitanmonolaurat + 20 Ethylenoxid (EO).
- Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten. Bevorzugte Vertreter dieser Klasse sind beispielsweise Nonylphenol + 4 EO, Nonylphenol + 9 EO, Octylphenol + 3 EO und Octylphenol + 8 EO.

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich erfindungsgemäße Mittel erwiesen, die nichtionogene grenzflächenaktive Substanzen in Mengen von 1 - 5 Gew.-% enthalten.

Weiterhin können die erfindungsgemäßen Blondiermittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.

Bevorzugte anionische Tenside sind Alkylsulfate, Ethercarbonsäuresalze mit C10 bis C18-Alkylgruppen und bis zu 12 Glykolethergruppen im Molekül wie C₁₂H₂₅-(C₂H₄O)₆-CH₂-COONa sowie insbesondere Salze von gesättigten und speziell ungesättigten C8 bis C22-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-%, vor.

Als nichtionische Tenside eignen sich insbesondere C8 bis C22-Alkylmono- und oligoglycoside und deren ethoxylierte Analoga. Insbesondere die nichtethoxylierten Verbindungen haben sich als besonders geeignet erwiesen.

Beispiele für die in den erfindungsgemäßen Blondiermitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B: Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, so genannte "Esterquats", wie das unter der Bezeichnung Dehyquart^{®}F 75 in Abmischung mit Cetearylalkohle erhältliche Distearoylethylhydroxyethylammoniummethosulfat.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Ferner können die erfindungsgemäßen Blondiermittel weitere Wirk-, Hilfs- und Zusatzstoffe, wie beispielsweise
- nichtionische Polymere wie beispielsweise Vinylpyrrolidinon/Vinylacrylat-Copolymere, Polyvinylpyrrolidinon und Vinylpyrrolidinon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidinon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose,Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- Wirkstoffe wie Panthenol, Pantothensäure, Allantoin, Pyrrolidoncarbonsäuren und deren Salze,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genusssäuren und Basen,
- Wirkstoffe wie Allantoin, Pyrrolidoncarbonsäuren und deren Salze sowie Bisabofol,
- Vitamine, Provitamine und Vitaminvorstufen, insbesondere solche der Gruppen A, B3, B5, B6, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch,

Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine, Fettalkohole und Fettsäureester,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N2O, Dimethylether, CO2 und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel können in einer Vielzahl von Angebotsformen bereitgestellt werden, beispielsweise in Form von Pasten, Pulvern, Tabletten usw., solange sie mindestens einen partikelförmigen Inhaltsstoff enthalten, welcher mit einer Hülle umgeben ist. Besonders bevorzugt liegen die erfindungsgemäßen Mittel im Hinblick auf die Anwendungskonvenienz jedoch als Blondierpulver vor. Erfindungsgemäße Mittel, die als Partikelgemisch ("in Pulverform") vorliegen, sind bevorzugte Ausführungsformen der vorliegenden Erfindung.

Es hat sich erfindungsgemäß als besonders vorteilhaft erwiesen, wenn die die beschichteten Partikel enthaltenden Mittel vor der Anwendung mit einer Oxidationslösung gemischt werden. Hierbei hat es sich insbesondere als vorteilhaft herausgestellt, wenn als eine Oxidationslösung eine Wasserstoffperoxid-Lösung eingesetzt.

Bevorzugte Wasserstoffperoxid-Lösung besitzen einen Gehalt von 0,1 bis 12 Gew.-%, insbesondere von 1,0 bis 8,0 Gew.-%.

Eine bevorzugte Darreichungsform der vorliegenden Erfindung stellt schließlich ein Kit-of-Parts dar, welches in einer gemeinsamen Verpackung getrennt voneinander in unterschiedlichen Behältern konfektioniert
i. ein Blondiermittel, enthaltend beschichtete Partikel gemäß der vorangegangenen Beschreibung und
ii. eine Wasserstoffperoxid-Lösung sowie
iii. gegebenenfalls eine Gebrauchsanleitung und/oder
iv. gegebenenfalls eine Applikationshilfe wie Bürste oder Pinsel
enthält.

Ein weiterer Gegenstand der vorliegenden Erfindung ist die Verwendung der erfindungsgemäßen Mittel zum Aufhellen von keratinischen Fasern, insbesondere von menschlichen Haaren.

Im ersten Schritt des erfindungsgemäßen Verfahrens wird mindestens ein partikelförmiger Inhaltsstoff mit mindestens einem der vorstehend genannten Stoffe beschichtet. Dieser Schritt lässt sich in den unterschiedlichsten Apparaturen problemlos durchführen.

Alternativ zum Einsatz eines Mischergranulators kann auch eine Wirbelschichtapparatur zur Ausbildung des Feststoffcoatings genutzt werden. Erfindungsgemäße Verfahren, bei denen die Beschichtung der zu beschichtenden Partikel in einer Wirbelschichtapparatur durchgeführt wird, sind bevorzugt.

Auch hier kann gleichzeitig Flüssigkeit auf die Körner aufgebracht werden. Die Beschichtung kann dabei gleichzeitig mit der Trocknung erfolgen (beispielsweise in einer Wirbelschichtapparatur, in der die Granulate mit einer Lösung oder Dispersion mindestens eines der oben genannten Stoffe beaufschlagt und gleichzeitig getrocknet werden), es ist aber auch möglich und bevorzugt, die Trocknung nach der Beschichtung, also zeitlich anschließend an diese, durchzuführen.

Es kann eine dichte Hülle erzeugt werden, indem eine Lösung oder Dispersion des/der oben genannten Stoffe(s) auf die zu beschichtenden Partikel aufgebracht wird. Diese Lösung bzw. Dispersion kann auch weitere filmbildende Substanzen enthalten. Ein erfindungsgemäßes Verfahren, bei dem die zu beschichtenden Partikel mit einer Lösung oder Dispersion eines Beschichtungsmittel, enthaltend mindestens eine modifizierte Cellulose gemäß Formel (I), besprüht und getrocknet werden, ist demnach eine weitere bevorzugte Ausführungsform der vorliegenden Erfindung.

Alternative Beschichtungsverfahren, die sich zur Herstellung erfindungsgemäßer Zusammensetzungen eignen, sind das Strahlschichtcoating sowie Schmelzextrusionsverfahren.

### Beispiele

Die nachfolgenden Beispiele sollen die vorliegende Erfindung erläutern, ohne sie zu begrenzen oder auf die Beispiele einzuschränken.

### Beispiel 1 Cellulose Acetat Phthalat (CAP) beschichtete Alkalisierungsmittel

**Tabelle 1**

| Rohstoff | Menge in g |
|---|---|
| Aquacoat^{®} CPD (30 Gew.-%)¹ | 750,0 |
| Talkum, mikron. | 112,5 |
| Triethylcitrat | 22,5 |
| Wasser | 500,0 |
| Ges. | 1385,0 |

| | |
|---|---|
| ¹Cellulose Acetat Phthalat (CAP), Firma FMC | |

Eine Coatingdispersion wird durch Vermischen der Zusammensetzung gemäß Tabelle 1 bei Raumtemperatur hergestellt.

Unter üblichen Bedingungen wurden 1230 g der Coatingdispersion gemäß Tabelle 1 mit Hilfe einer Wirbelschichtapparatur auf 500 g Portil^{®} A (Natriumsilikat, Firma Cognis GmbH, Deutschland) aufgebracht.

Nach dem Trocknen verblieben Partikel mit Beschichtung, wobei der Gewichtsanteil der Hülle bei 39 Gew.-%, bezogen auf das Partikelgesamtgewicht, liegt. Der Anteil des Coating-Polymers innerhalb der Hülle entspricht 63 Gew.-%, bezogen auf das Gesamtgewicht der Beschichtung.

### Beispiel 2 Ethylcellulose beschichtete Alkalisierungsmittel (nicht erfindungsgemäß)

**Tabelle 2a**

| Rohstoff | Menge in g |
|---|---|
| Kollidon^{®} CL-M¹ | 30 |
| Wasser | 600 |

| | |
|---|---|
| ¹quervernetztes Polyvinylpyrrolidinon, unlöslich, Firma BASF | |

**Tabelle 2b**

| Rohstoff | Menge in g |
|---|---|
| Surelease^{®} (25 Gew.-%)² | 850 |
| Wasser | 600 |

| | |
|---|---|
| ²Ethylcellulose-Dispersion, Firma Colorcon | |

Eine Coatingdispersion wird durch Vermischen der Zusammensetzung gemäß Tabelle 2 bei Raumtemperatur hergestellt.

Wie unter Beispiel 1 wurden nacheinander zunächst 600 g einer Coatingdispersion gemäß Tabelle 2a mit Hilfe einer Wirbelschichtapparatur auf 500 g Portil^{®} A (Natriumsilikat, Firma Cognis GmbH, Deutschland) aufgebracht und anschließend getrocknet.

Daraufhin wurden 1365 g einer Coatingdispersion gemäß Tabelle 2b mit Hilfe einer Wirbelschichtapparatur auf die bereits beschichteten Portil^{®} A-Partikel aufgebracht.

Nach dem Trocknen verblieben Partikel mit Beschichtung, wobei der Gewichtsanteil der Hülle bei 31 Gew.-%, bezogen auf das Partikelgesamtgewicht, liegt. Der Anteil des Coating-Polymers innerhalb der Hülle entspricht 88 Gew.-%, bezogen auf das Gesamtgewicht der Beschichtung.

### Beispiel 3 Blondierpulver

Die in den Beispielen 1 und 2 beschriebenen, umhüllten Partikel wurden in Blondierpulvermischungen eingesetzt (Tabelle 3).

| Rohstoffe und Handelsprodukte | V 1 (Mengen in g) | E 1 (Mengen in g) | V 2 (Mengen in g) |
|---|---|---|---|
| Magnesiumcarbonat | 28,380 | 28,380 | 28,380 |
| Natriumhexametaphosphat | 0,231 | 0,231 | 0,231 |
| Rohagit^{®} S hv ¹ | 2,077 | 2,077 | 2,077 |
| Na₂EDTA | 0,692 | 0,692 | 0,692 |
| Celquat^{®} L-200² | 0,385 | 0,385 | 0,385 |
| Glycin | 0,692 | 0,692 | 0,692 |
| Natriumpersulfat | 18,000 | 18,000 | 18,000 |
| Ammoniumpersulfat + 0,5% Kieselsäure | 12,000 | 12,000 | 12,000 |
| Ariabel^{®} Blue³ | 0,154 | 0,154 | 0,154 |
| Dow Corning ^{®} 1403 Fluid⁴ | 1,769 | 1,769 | 1,769 |
| Parfum | 0,692 | 0,692 | 0,692 |
| Paraffinum Liquidum | 11,850 | 11,850 | 11,850 |
| Natriumsilikat (Portil^{®} A)⁵ | 30,000 | - | - |
| Natriumsilikat-Partikel gemäß Beispiel 1 | - | 49,180 | - |
| Natriumsilikat-Partikel gemäß Beispiel 2 | - | - | 43,478 |
| gesamt | **106,922** | **126,102** | **120,400** |

| | | | |
|---|---|---|---|
| ¹Methylmethacrylat-Methacrylsäure Copolymer (INCI-Bezeichnung: Acrylates Copolymer; Firma Evonik Röhm, Deutschland) ²quaternisiertes Cellulosederivat (INCI-Bezeichnung: Polyquaternium-4; Firma National Starch) ³Pigment Blue 29 (INCI-Bezeichnung: Cl 77007, Ultramarines; Firma Golmann-Warner Jenkinson) ⁴Silikon (INCI-Bezeichnung: Dimethicone, Dimethiconol, Firma Dow Corning) ⁵Natriumsilikat, Firma Cognis GmbH, Deutschland | | | |

Die jeweiligen Blondierpulver werden vor der Anwendung zu gleichen Gewichtsteilen mit einer 6 Gew.-% Wasserstoffperoxid-Lösung vermischt.

## Patentansprüche

1. Mittel zum Aufhellen von keratinischen Fasern, insbesondere menschlicher Haare, enthaltend mindestens einen umhüllten Partikel und eine Oxidationsmittellösung, vorzugsweise eine Wasserstoffperoxidlösung, wobei die Partikel einen festen Partikelkern, ausgewählt aus Alkalimetall-silikaten, umfassen und eine diesen Kern umgebende Hülle beinhalten, **dadurch gekennzeichnet, dass** die Hülle zu mindestens 50 Gew.-% ihres Gewichts aus einer modifizierten Cellulose mit Monomereinheiten entsprechend Formel (I) 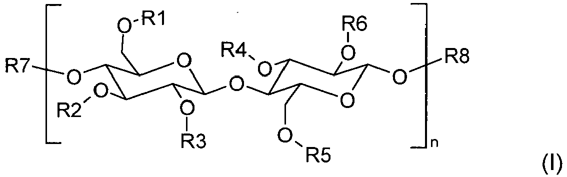 besteht,
worin R1 bis R8 unabhängig voneinander für Wasserstoff, einen linearen oder verzweigten C1 bis C6-Alkylrest, einen linearen oder verzweigten C1 bis C6-Hydroxyalkylrest, enthaltend eine oder mehrere Hydroxylgruppen, einen linearen oder verzweigten C1 bis C6-Alkoxyalkylrest, enthaltend eine oder mehrere C1 bis C4-Alkoxygruppen, einen linearen oder verzweigten C1 bis C6-Acylrest oder einen gegebenenfalls substituierten Benzoylrest steht, mit der Maßgabe, dass
- mindestens einer der Reste R1 bis R6 ungleich Wasserstoff ist,
- R1 bis R6 unabhängig voneinander für Wasserstoff, einen Acetylrest oder einen Phthalatrest steht,
- mindestens einer der Reste R1 bis R6 für einen Acetylrest und mindestens einer der Reste R1 bis R6 für einen Phthalatrest steht
- und n für eine ganze Zahl zwischen 10 und 1.000.000 steht.

2. Mittel nach Anspruch 1, **dadurch gekennzeichnet, dass** die den Partikelkern umgebende Hülle zusätzlich mindestens ein Desintegrationsmittel enthält.

3. Mittel nach einem der Ansprüche 1 bis 2, **dadurch gekennzeichnet, dass** die den Partikelkern umgebende Hülle zusätzlich mindestens ein Trennmittel und/oder mindestens einen Porenbildner enthält.

4. Verwendung eines Mittels gemäß einem der Ansprüche 1 bis 3 zum Aufhellen von keratinischen Fasern, insbesondere menschlichen Haare.

## Claims

1. An agent for lightening keratin fibers, in particular human hair, containing at least one encased particle and an oxidizing agent solution, preferably a hydrogen peroxide solution, the particles having a solid particle core selected from alkali metal silicates and containing a shell surrounding this core, **characterized in that** the shell consists of a modified cellulose having monomer units corresponding to formula (I) 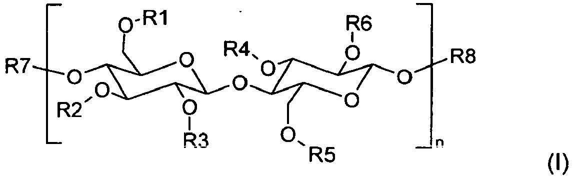 in a proportion of at least 50 wt.% of its weight,
where R1 to R8, independently of one another, represent hydrogen, a linear or branched C1 to C6 alkyl functional group, a linear or branched C1 to C6 hydroxyalkyl functional group containing one or more hydroxyl groups, a linear or branched C1 to C6 alkoxyalkyl functional group containing one or more C1 to C4 alkoxy groups, a linear or branched C1 to C6 acyl functional group or an optionally substituted benzoyl functional group, providing that
- at least one of the functional groups R1 to R6 is not hydrogen,
- R1 to R6, independently of one another, represent hydrogen, an acetyl functional group or a phthalate functional group,
- at least one of the functional groups R1 to R6 represents an acetyl functional group and at least one of the functional groups R1 to R6 represents a phthalate functional group,
- and n represents an integer of between and 10 and 1,000,000.

2. The agent according to claim 1, **characterized in that** the shell surrounding the particle core additionally contains at least one disintegration agent.

3. The agent according to one of claims 1 to 2, **characterized in that** the shell surrounding the particle core additionally contains at least one release agent and/or at least one pore former.

4. The use of an agent according to one of claims 1 to 3 for lightening keratin fibers, in particular human hair.

## Revendications

1. Produit pour éclaircir des fibres de kératine, en particulier des cheveux humains, contenant au moins une particule enveloppée et une solution d'oxydant, préférentiellement une solution de peroxyde d'hydrogène, les particules ayant un noyau solide choisi parmi des silicates de métaux alcalins, et une enveloppe entourant ce noyau, **caractérisé en ce que** l'enveloppe est constituée d'au moins 50 % en poids, rapporté à son poids, d'une cellulose modifiée avec des unités monomères correspondant à la formule (I) 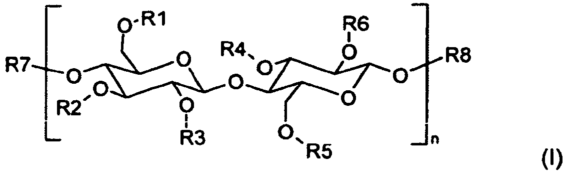 où
R1 à R8 représentent, indépendamment les uns des autres, un hydrogène, un résidu C₁₋₆-alkyle linéaire ou ramifié, un résidu C₁₋₆-hydroxyalkyle linéaire ou ramifié contenant un ou plusieurs groupes hydroxyles, un résidu C₁₋₆-alcoxyalkyle linéaire ou ramifié contenant un ou plusieurs groupes C₁₋₄-alcoxy, un résidu C₁₋₆-acyle linéaire ou ramifié, ou un résidu benzoyle éventuellement substitué, à condition que :
- au moins un des résidus R1 à R6 ne soit pas un hydrogène,
- R1 à R6 représentent, indépendamment les uns des autres, un résidu acétyle ou un résidu phtalate,
- au moins un des résidus R1 à R6 représente un résidu acétyle et au moins un des résidus R1 à R6 représente un résidu phtalate
- n est un entier entre 10 et 1 000 000.

2. Produit selon la revendication 1, **caractérisé en ce que** l'enveloppe qui entoure les particules contient en plus au moins un agent de désintégration.

3. Produit selon la revendication 1 ou 2, **caractérisé en ce que** l'enveloppe qui entoure les particules contient en plus au moins un agent de démoulage et/ou au moins un agent de formation de pores.

4. Utilisation d'un produit selon une des revendications 1 à 3 pour éclaircir des fibres de kératine, en particulier des cheveux humains.
